# EUROPÄISCHE PATENTSCHRIFT

(11) **EP 0 467 337 B1**
(45) Veröffentlichungstag und Bekanntmachung des Hinweises auf die Patenterteilung: **04.06.1997**
(21) Anmeldenummer: 91111961.8
(22) Anmeldetag: 17.07.1991
(51) Int. Cl.: A01N 43/80, A01N 43/40, A01N 47/36, A01N 43/28, G01N 33/50

(54) **Konservierung von diagnostischen Tests**
Conservation of diagnostic tests
Conservation des essais diagnostiques

(30) Priorität: 18.07.1990 DE 4022878
(43) Veröffentlichungstag der Anmeldung: 22.01.1992
(73) Patentinhaber: BOEHRINGER MANNHEIM GMBH, 68298 Mannheim (DE)
(72) Erfinder: Hoss, Gerhard, Dr. rer. nat., W-8120 Weilheim (DE); Pappert, Dr. rer. nat., W-8132 Tutzing (DE); Schmidt, Axel, Dr. rer. nat., W-8000 München 19 (DE)
(74) Vertreter: Huber, Bernhard, Dipl.-Chem.

(56) Entgegenhaltungen:
- EP-A- 0 029 917
- EP-A- 0 067 394
- EP-A- 0 375 367
- FR-A- 2 345 164
- CHEMICAL ABSTRACTS, vol. 108, no. 21, 23. Mai 1988, Columbus, Ohio, US; M Terajima. Seite 378
- EP 398795
- EP 194466

## Beschreibung

Die Erfindung betrifft die Konservierung diagnostischer Tests, insbesondere Testkits mittels einer Kombination von Konservierungsmitteln sowie diagnostische Tests, die solche Mittel enthalten.

Biologische, insbesondere diagnostische Testkits enthalten üblicherweise als Reagenzien und als Standards Substanzen, die durch Mikroorganismen zersetzbar sind. Zur Verbesserung ihrer Haltbarkeit müssen diese daher vom Hersteller konserviert werden. Es hat sich jedoch bislang als äußerst schwierig erwiesen, geeignete Konservierungsmittel für solche Diagnostika aufzufinden, da neben einer ausreichenden Wirksamkeit gegenüber Bakterien, Hefen und Pilzen die Reaktivität von im Test vorhandenen Substanzen durch das verwendete Konservierungsmittel nicht gestört werden darf. Die Empfindlichkeit von Proteinen wie z.B. Enzymen oder Antikörpern sowie von Antigenen und Substraten gegenüber Konservierungsmitteln zeigt sich z.B. darin, daß die Proteine denaturiert werden können oder auch immunogene Strukturen zerstört werden. Darüber hinaus binden zugesetzte Konservierungsmittel häufig solche Substanzen und stellen dadurch eine Konkurrenz zum eigentlichen Bindungspartner im Test dar (z.B. Bindung Enzym/Substrat, Antikörper/Antigen). Bislang war es daher notwendig, für einen bestimmten diagnostischen Test jeweils ein genau dafür geeignetes Konservierungsmittel aufzufinden, welches die zuvor genannten Eigenschaften aufweist.

Die EP-A-0 067 394 offenbart eine wäßrige Cholesterin-Standardlösung, die ein Detergenzgemisch und gegebenenfalls zusätzlich geeignete Konservierungsmittel wie Azid, Germal oder Chloracetamid und einen Puffer enthält. Durch die Verwendung der Detergentien Cholsäure und Desoxycholsäure wurde eine alkoholfreie, stabile, wäßrige Cholesterinstandardlösung erhalten, die in ihrem kinetischen Verhalten bei der Cholesterinbestimmung den zu messenden Cholesterinproben gleicht.

Die Erfindung hat nun zum Ziel, Konservierungsmittel bereitzustellen, die ganz allgemein bei diagnostischen Tests verwendbar sind, in ihrer konservierenden Eigenschaft ein großes Keimspektrum abdecken und die Bildung von resistenten Keimen minimieren. Auf diese Weise soll die Prüfung und Lagerhaltung von Konservierungsmitteln auf wenige Substanzen beschränkt werden und die Suche nach neuen Konservierungsmitteln, welche die zuvor geschilderten Eigenschaften aufweisen, verringert werden bzw. ganz entfallen.

Gelöst wird diese Aufgabe durch eine Kombination von Konservierungsmitteln, die sich im besonderen zum Konservieren von diagnostischen Tests eignet und die einen Gehalt an mindestens zwei Komponenten, ausgewählt aus der Gruppe, umfassend 2-Methyl-4-isothiazolin-3-on-hydrochlorid, 2-Hydroxpyridin-N-oxid, Chloracetamid, (N,N-methylen-bis(N-(1-hydroxymethyl)-2,5-dioxo-4-imidazolidinyl))-harnstoff und 5-Brom-5-nitro-1,3-dioxan, ausgenommen die Kombination von Chloracetamid mit (N,N-methylen-bis(N-1-hydroxymethyl)-2,5-dioxo-4-imidazolidinyl))-harnstoff, aufweist.

Es hat sich nämlich überraschenderweise gezeigt, daß die erfindungsgemäße Kombination von mindestens zwei der zuvor genannten Substanzen gegen ein großes Spektrum verschiedenster Keime nicht nur biozid wirkt, sondern dabei auch gleichzeitig keinen negativen Einfluß auf die Ergebnisse von diagnostischen Testverfahren hat.

Besonders bevorzugt ist es, 2-Methyl-4-isothiazolin-3-on-hydrochlorid in Kombination mit mindestens einem der zuvor genannten Konservierungsmittel zu verwenden. Besonders bevorzugt sind daher Konservierungsmittel, die 2-Methyl-4-isothiazolin-3-on-hydrochlorid und 2-Hydroxypyridin-N-oxid bzw. 2-Methyl-4-isothiazolin-3-on-hydrochlorid und Chloracetamid bzw. 2-Methyl-4-isothiazolin-3-on-hydrochlorid und N,N-Methylen-bis(N-(1-hydroxymethyl)-2,5-dioxo-4-imidazolidinyl))-harnstoff oder 2-Methyl-4-isothiazolin-3-on-hydrochlorid und 5-Brom-5-nitro-1,3-dioxan enthalten.

Ebenso ist es bevorzugt 5-Brom-5-nitro-1,3-dioxan mit jeweils einem anderen der genannten Konservierungsmittel zu kombinieren.

2-Methyl-4-isothiazolin-3-on-hydrochlorid (im weiteren auch als Methylisothiazolin bezeichnet) ist nach den Verfahren erhältlich, wie sie von S. N. Lewis et al., J. Heterocycl. Chem. (1971), 8, 571 und in der US-PS 4,105,431 (1968) beschrieben sind. 2-Hydroxypyridin-N-oxid wird als 20%ige Lösung unter dem Namen Oxy-pyrion von Pyrion-Chemie, Neuss, Bundesrepublik Deutschland vertrieben und läßt sich daraus durch eine Extraktion mit Essigsäureethylester und/oder Chloroform und anschließender Entfernung des Extraktionsmittels durch Destillation isolieren. (N,N-methylen-bis-(N-(1-hydroxymethyl)-2,5-dioxo-4-imidazolidinyl))-harnstoff wird unter dem Handelsnamen Germall 115 von Chemag AG, Frankfurt, Bundesrepublik Deutschland, vertrieben. 2-Chlorazetamid ist von E. Merck, Darmstadt, Bundesrepublik Deutschland erhältlich. 5-Brom-5-nitro-1,3-dioxan wird von Henkel KG, Bundesrepublik Deutschland, unter dem Handelsnamen BRONIDOX vertrieben.

Die antimikrobielle Wirksamkeit dieser Substanzen ist für einzelne oder mehrere Mikroorganismen bekannt. Es ist jedoch überraschend, daß eine Kombination davon die hochempfindlichen Reaktionsmechanismen, wie sie bei biologischen, insbesondere diagnostischen Testverfahren stattfinden, ganz allgemein nicht beeinträchtigen.

Die Erfindung betrifft daher auch diagnostische Tests, insbesondere diagnostische Testkits, die als Konservierungsmittel eine Kombination aus mindestens zwei Substanzen, ausgewählt aus der Gruppe 2-Methyl-4-isothiazolin-3-on-hydrochlorid, 2-Hydroxypyridin-N-oxid, Chloracetamid und (N,N-methylen-bis(N-(1-hydroxymethyl)-2,5-dioxo-4- imidazolidinyl))-harnstoff und 5-Brom-5-nitro-1,3-dioxan enthalten.

In den erfindungsgemäß konservierten Tests sind 2-Methyl-4-isothiazolin-3-on-hydrochlorid in einer Menge von 0,001 bis 0,5 %, 2-Hydroxy-pyridin-N-oxid, Chlorazetamid und (N,N-methylen-bis-(N-(1-hydroxymethyl)-2,5-dioxo-4-imidazolidinyl))-harnstoff in einer Menge von 0,1 bis 0,5 % und 5-Brom-5-nitro-1,3-dioxan in einer Menge von 0,001 % bis 0,5 % enthalten, bezogen auf die Konzentration in der wäßrigen Lösung, in welcher der Testkit bzw. die Standards oder Pufferlösungen bis zu ihrer Verwendung gelagert werden. Es hat sich gezeigt, daß die erfindungsgemäße Konservierungsmittel-Kombination im Testkit sowohl dem Puffer als auch dem jeweiligen Testreagenz zugesetzt werden kann. Es hat sich jedoch auch als zweckmäßig erwiesen, die Konservierungsmittel-Kombination den Testreagenzien und den Pufferlösungen gleichzeitig zuzusetzen. Bei den Testreagenzien in den erfindungsgemäßen Testkits handelt es sich üblicherweise um biologisch aktive Proteine und insbesondere um Enzyme und/oder Antikörper.

Die Erfindung betrifft auch die Verwendung der erfindungsgemäßen Konservierungsmittel-Kombination zum Konservieren von diagnostischen Tests.

Die erfindungsgemäße Konservierungsmittel-Kombination hat sich für solche Tests als ganz besonders geeignet erwiesen, die Proteine, insbesondere Enzyme, Antikörper und/oder Proteinhormone, Substrate für solche Enzyme, Hormone, insbesondere T₃, T₄ bzw. Steroide und/oder Antigene bzw. Haptene enthalten.

Die Erfindung soll durch die folgenden Beispiele näher erläutert werden.

### Beispiel 1

1.1 Herstellung der Mischsuspension "Bakterien"
   Escherichia coli ATCC 8739
   Pseudomonas aeruginosa ATCC 9027
   Staphylococus aureus ATCC 6538 P
   werden mittels Impföse auf CaSo-Agar ausgestrichen und 16 - 24 h bei 31°C bebrütet. Aus der Zone des Impfstrichs, wo konfluentes Wachstum in Einzelkolonien übergeht, wird mittels Impföse von mindestens 5 Kolonien stammendes Bakterienmaterial abgenommen und in ca. 10 ml physiologischer Kochsalzlösung homogen suspendiert. Die so erhaltene Suspension wird als Testkeimsuspension T bezeichnet.
   Die Suspension T wird mit physiologischer Kochsalzlösung so verdünnt, daß 10 ml Impfsuspension mit der Koloniezahl 10⁷ KBE/ml resultieren. Vom Bacillus subtilis wird eine Sporensuspension in destilliertem Wasser vorrätig gehalten, deren Titer in vierteljährigem Abstand neu bestimmt wird. Zur Herstellung der Mischsuspension "Bakterien" werden die einzelnen Impfsuspensionen zu gleichen Teilen gemischt. Dieser Mischsuspension wird soviel Sporensuspension Bacillus subtilis zugesetzt, daß 10⁵ Sporen pro ml resultieren. Diese Suspension wird als Mischsuspension "Bakterien" bezeichnet.
2.2 Mischsuspension " Schimmelpilze und Hefen"
   Candida albicans ATCC 10231
   Rhodotorula rubra DSM 70403
   Aspergillus oryzae DSM 6303
   Mucor racemosus ATCC 7935
   Penicillium frequentans DSM 62843
   werden auf Schrägagarröhrchen mit Sabouraud-Maltose-Agar geimpft und 12 - 16 Tage bei 23°C bebrütet. Die Kulturen werden mit ca. 2 ml physiologischer Kochsalzlösung überschichtet und mit Hilfe eines sterilen Glasstabes aufgeschwemmt. Die so entstandene Suspension wird mit einer sterilen physiologischen Kochsalzlösung so verdünnt, daß die Trübung dem Mc-Farland-Standard 10⁸ entspricht. Diese Suspension wird mit steriler physiologischer Kochsalzlösung 1 : 10 verdünnt, woraus eine Suspension von ca. 10⁷ Sporen oder Hefezellen pro ml resultiert. Diese Suspension wird als Mischsuspension "Schimmelpilze und Hefen" bezeichnet.

### Beispiel 2

Mit den nach Beispiel 1 hergestellten Mischsuspensionen wurden die in Tabelle 1 angegebenen Komponenten von im Handel erhältlichen Testkitlösungen untersucht.

### Ansatz A

Pro 50 ml der zu untersuchenden Lösung werden 0.5 ml Mischsuspension "Bakterien" zugegeben. Der Ansatz enthält somit ca 10⁵ KBE/ml, wobei der Anteil des Bacillus subtilis ca. 10³ KBE/ml beträgt. Die Lagertemperatur des Ansatzes ist 31°C.

### Ansatz B

Pro 50 ml der zu untersuchenden Lösung werden 0.5 ml Mischsuspension "Schimmelpilze und Hefen" zugegeben. Der Ansatz enthält somit ca 10⁵ KBE/ml Schimmelpilze und Hefen. Die Lagertemperatur des Ansatzes ist 23°C.

Nach einer Lagerzeit von 6 Wochen wird die Koloniezahl der Ansätze A und B bestimmt.
Hierzu werden Proben von 0.5 - 2 ml aus den Ansätzen entnommen und in 2 parallelen Verdünnungsreihen mit physiologischer Kochsalzlösung, entsprechend dem zu erwartenden Keimtiter, verdünnt und durch Ausplattieren auf Nähragarplatten die Koloniezahl bestimmt. Es werden jeweils 0.1 ml manuell oder mittels Spiralplattenapparat auf Standardagarplatten (90 mm ⌀) verteilt. Die Verdünnung hängt von der zu erwartenden Koloniezahl ab.

Die Ergebnisse sind in der Tabelle 1 angegeben. Die Werte in der Tabelle geben an, um welche Anzahl sich die Keime durch Zusatz des Konservierungsmittels nach der Lagerzeit von 6 Wochen vermindern. Die jeweiligen in der Tabelle angegebenen Bestellnummern geben die Bestellnummern der Firma Boehringer Mannheim GmbH, Bundesrepublik Deutschland an.

Die in den Tabellen verwendeten Abkürzungen lauten wie folgt:
- Brij 35®: : Tensid auf Basis von Polyethylenglycolethern des Laurylalkohols (Hersteller: Atlas Chemie)
- POD: : Peroxidase
- AFP: : α-Fetoprotein
- CEA: : Carcinoembryonales Antigen
- FSH: : Follikel stimulierendes Hormon
- HCG: : Humanes Choriogonadotropin
- LM: : Luteinisierendes Hormon
- MES: : 4-Morpholinethansulfonsäure-Puffer
- Anti-Progesteron-biotin:: Konjugat aus Biotin und einem Antikörper gegen Progesteron
- PEG 40000/6000: : Polyethylenglykol mit einem mittleren Molekulargewicht von 40000/6000
- Anti-ApoAI: : Antikörper gegen Apolipoprotein AI
- Anti-ApoB: : Antikörper gegen Apolipoprotein B

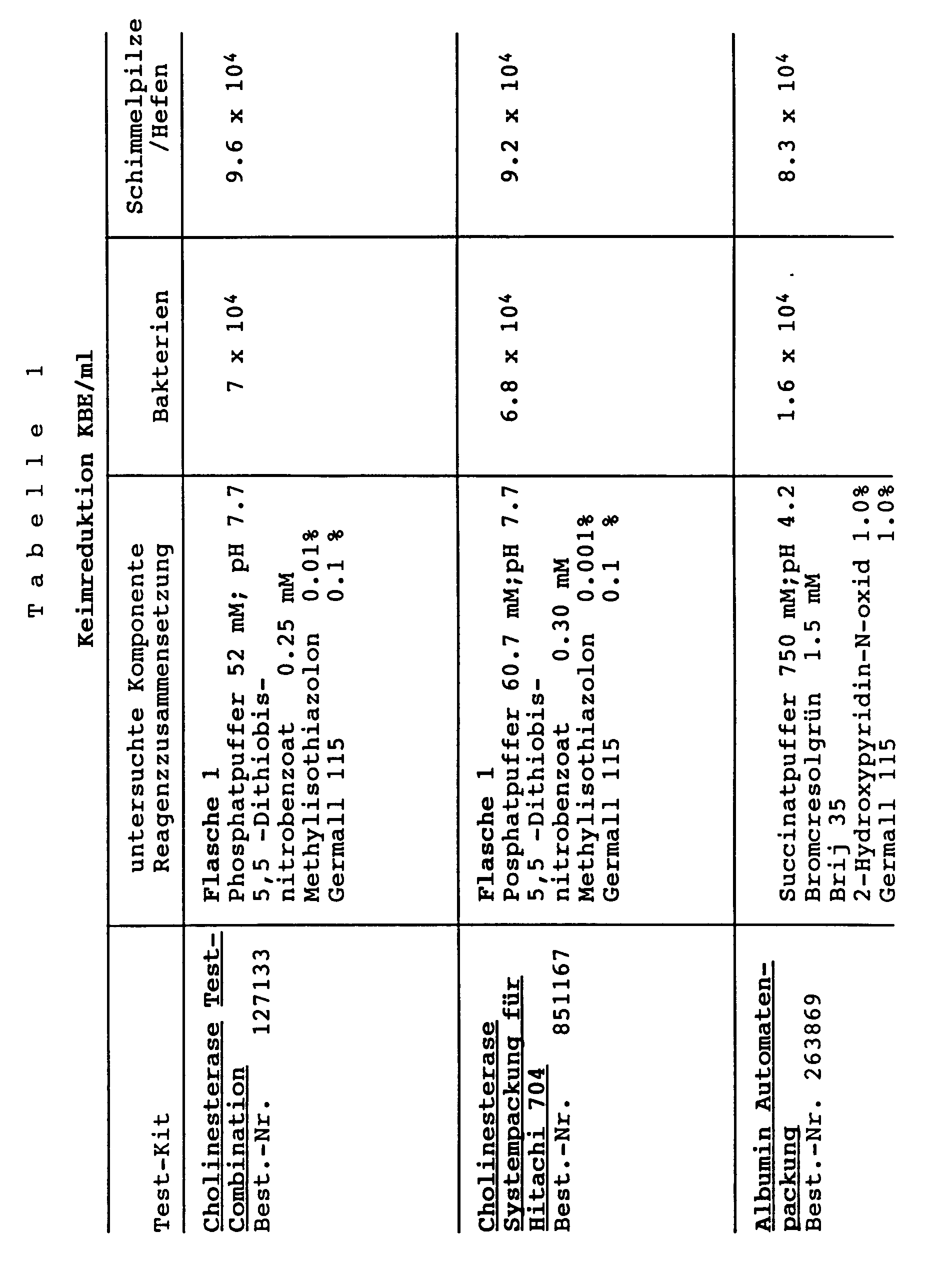

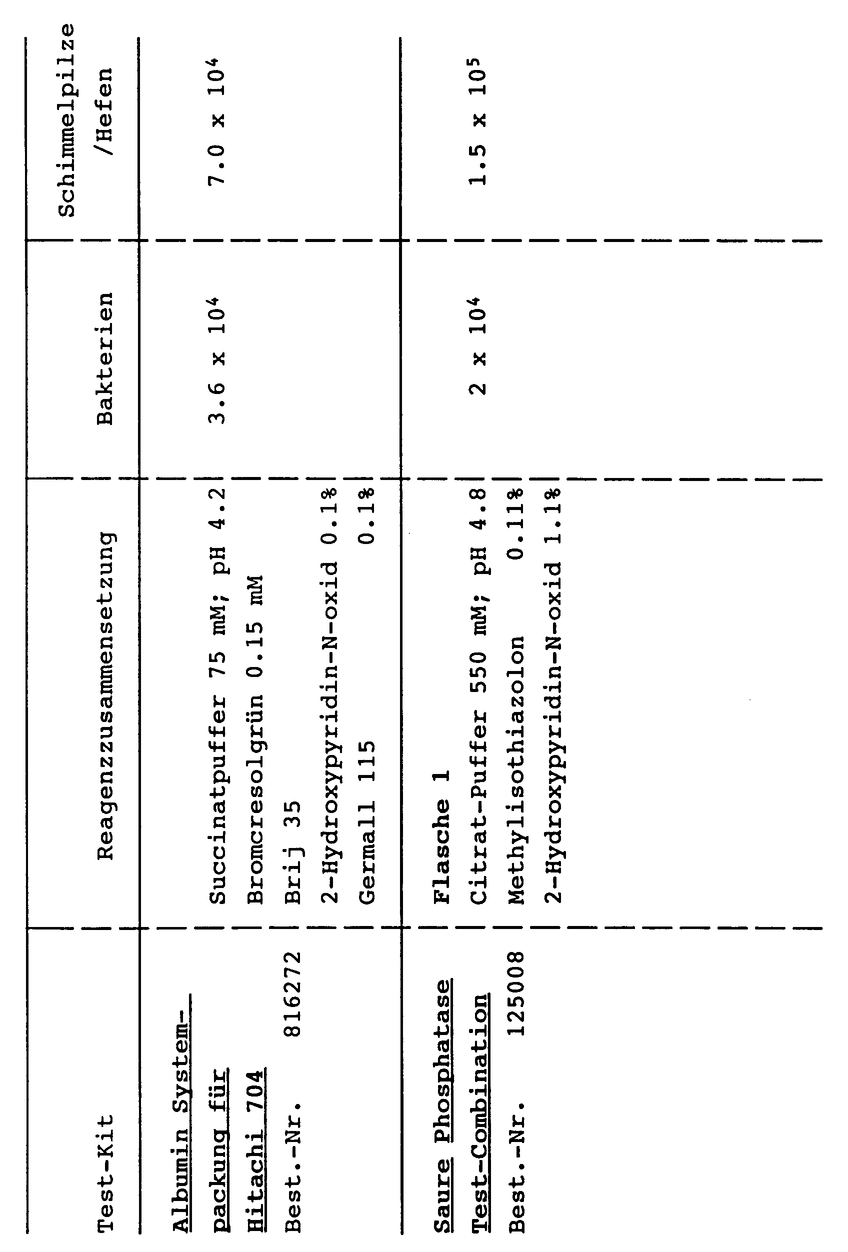

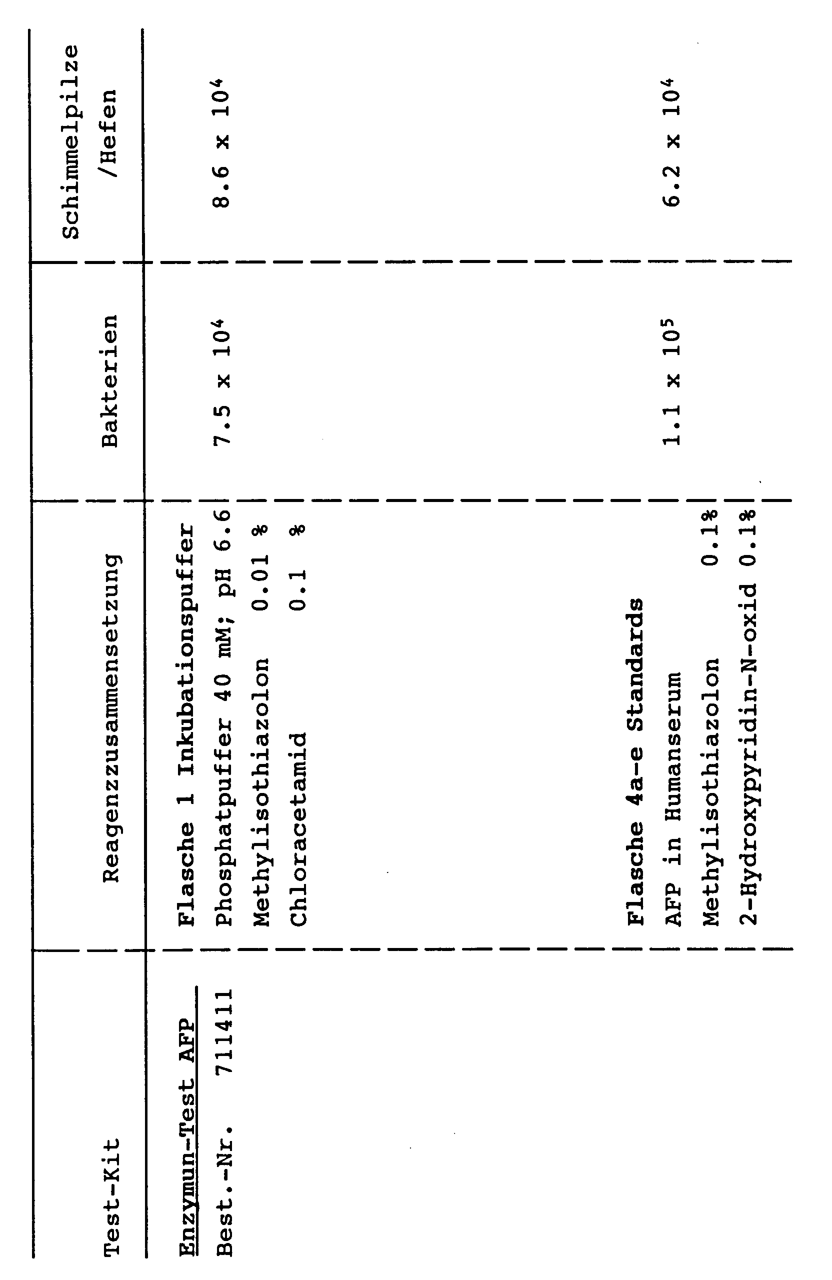

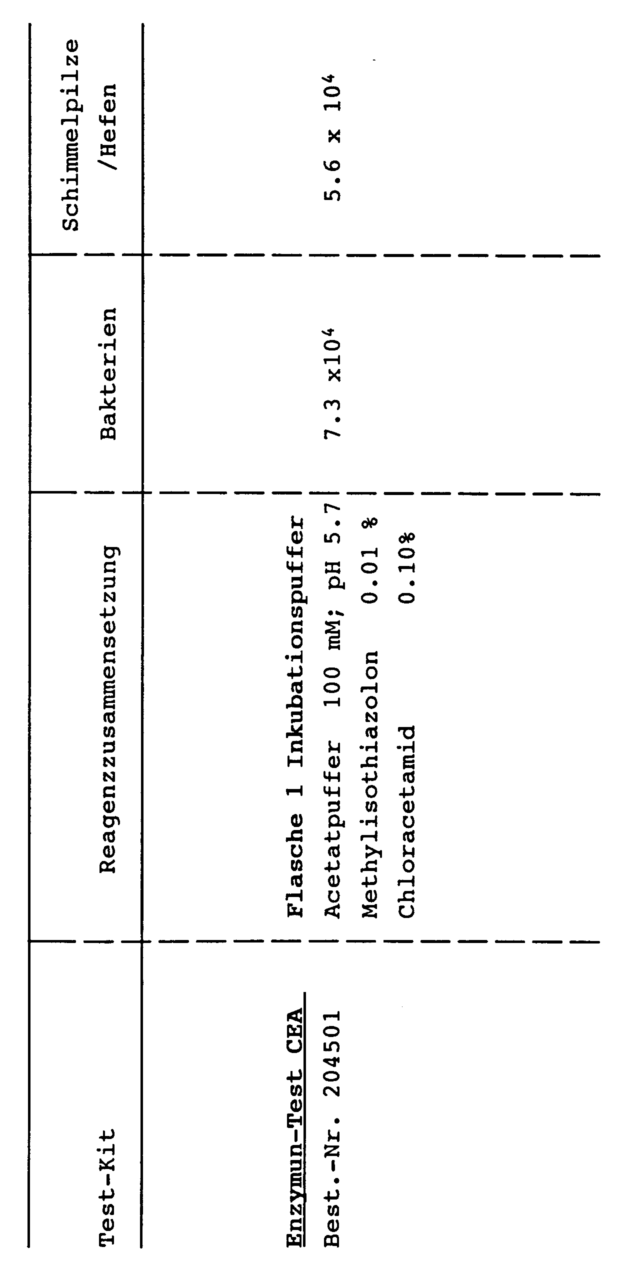

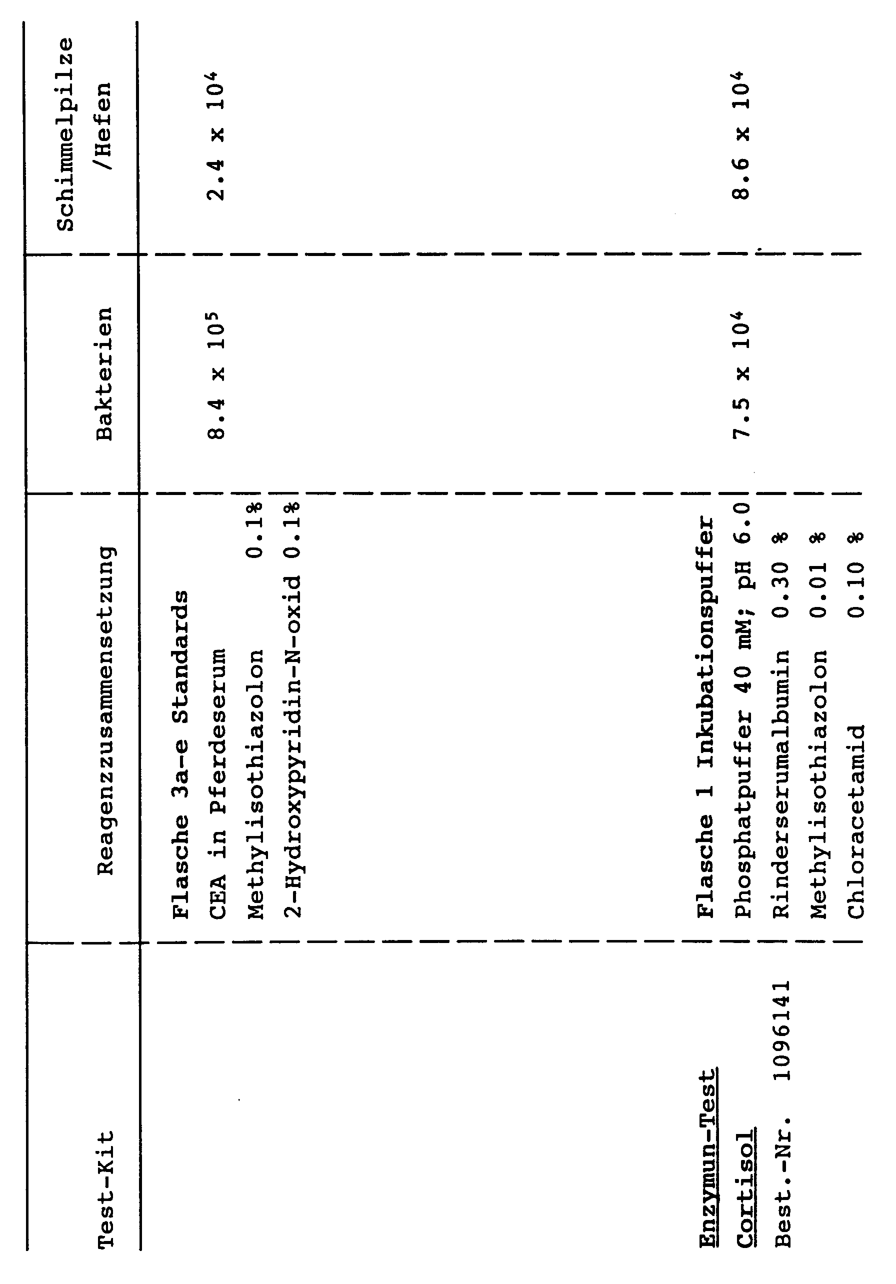

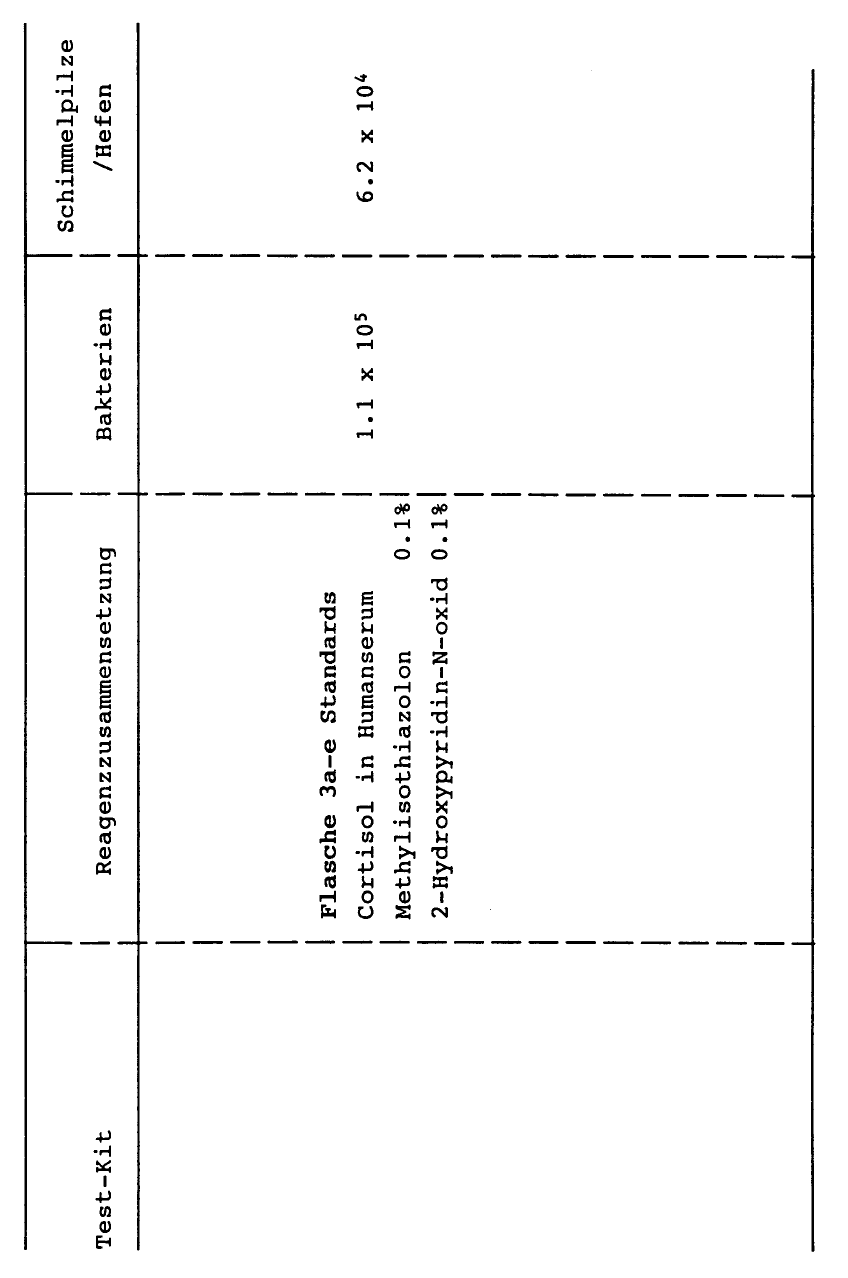

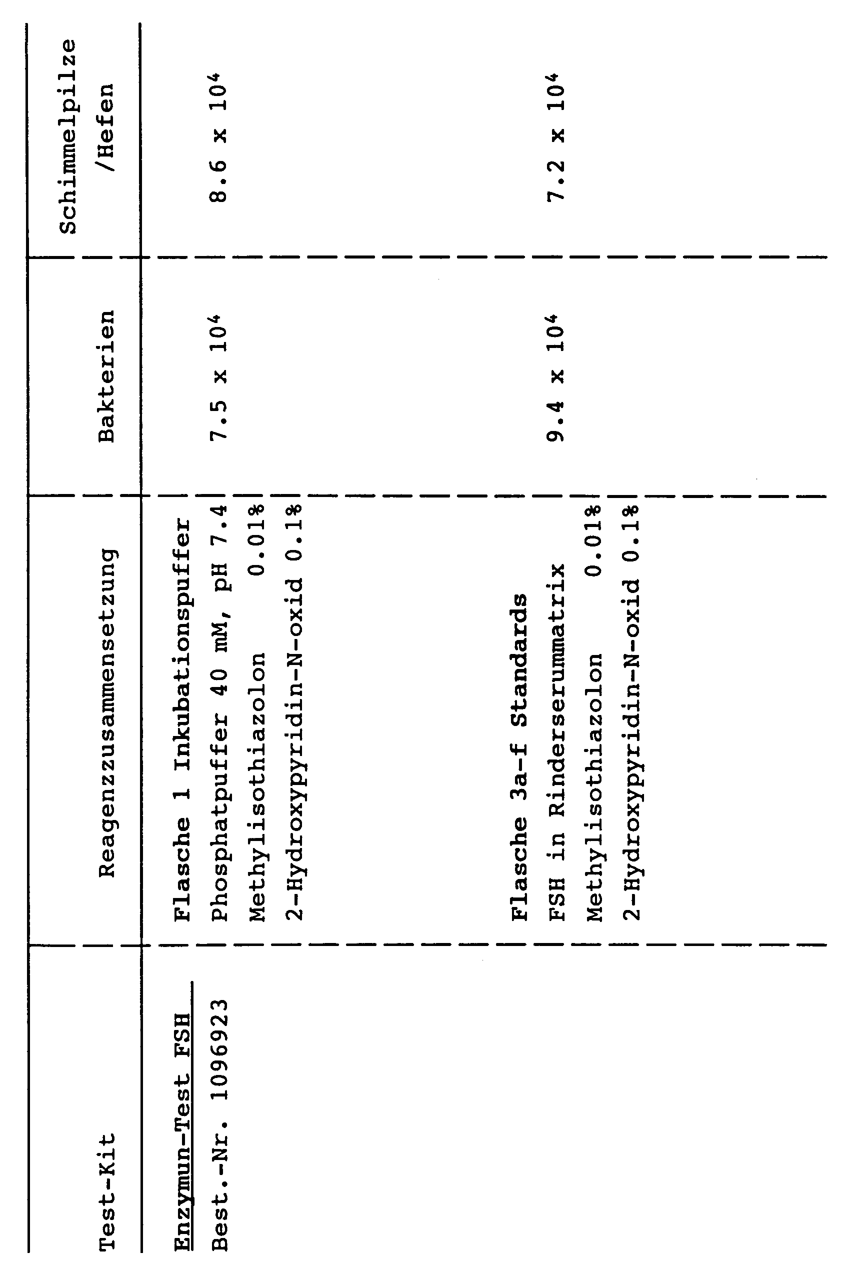

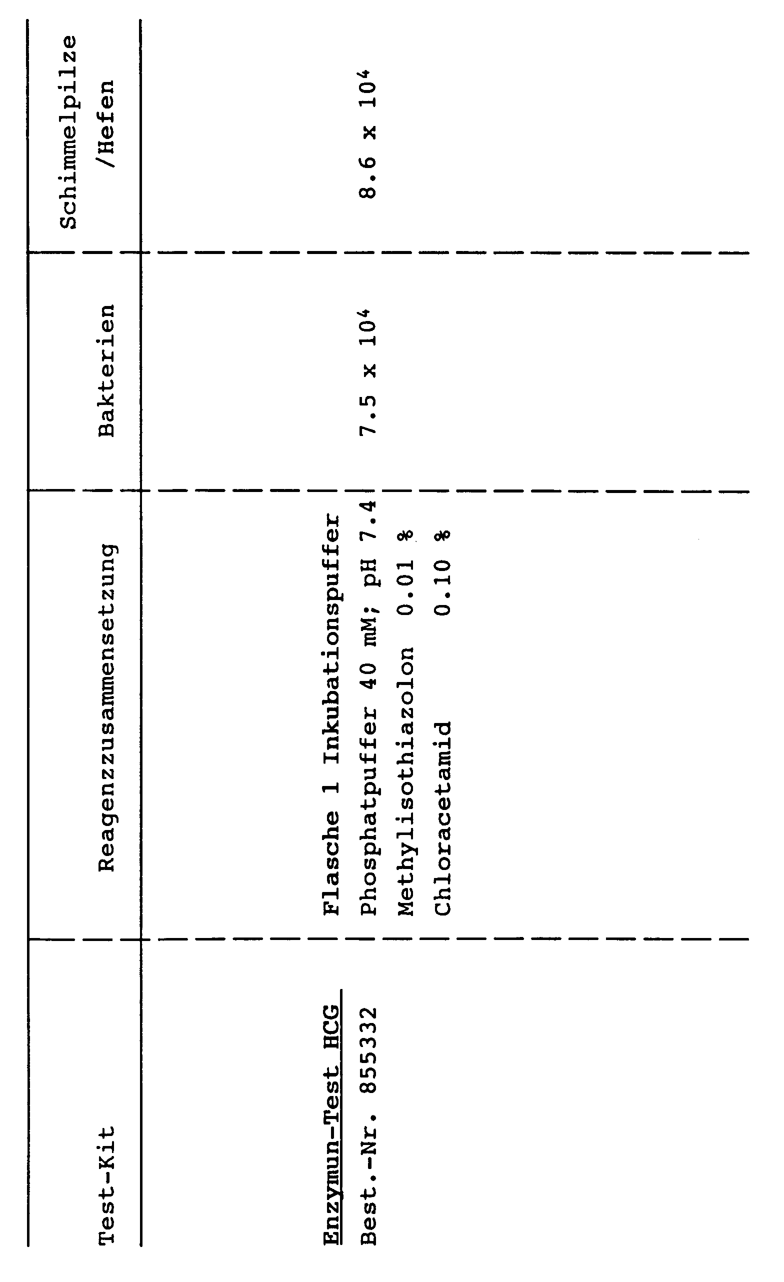

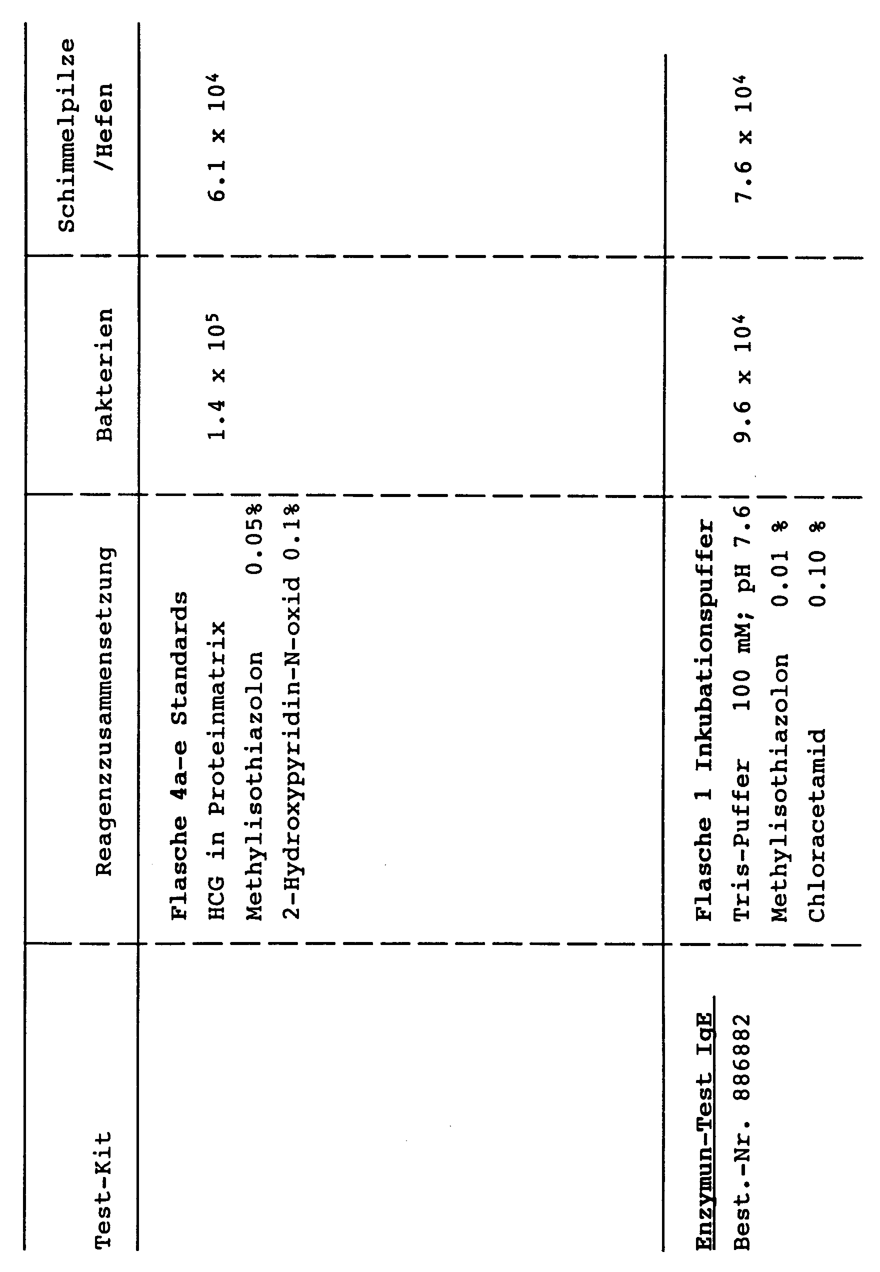

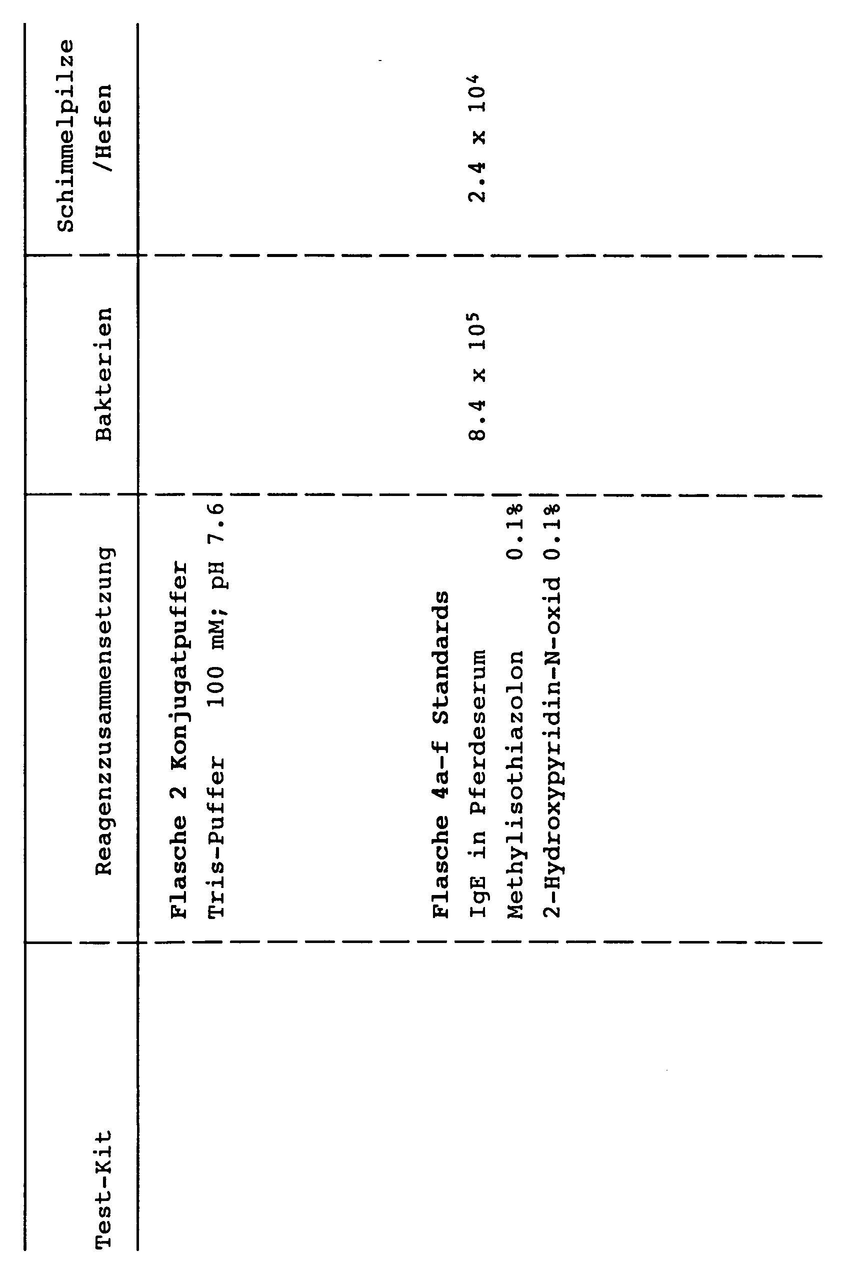

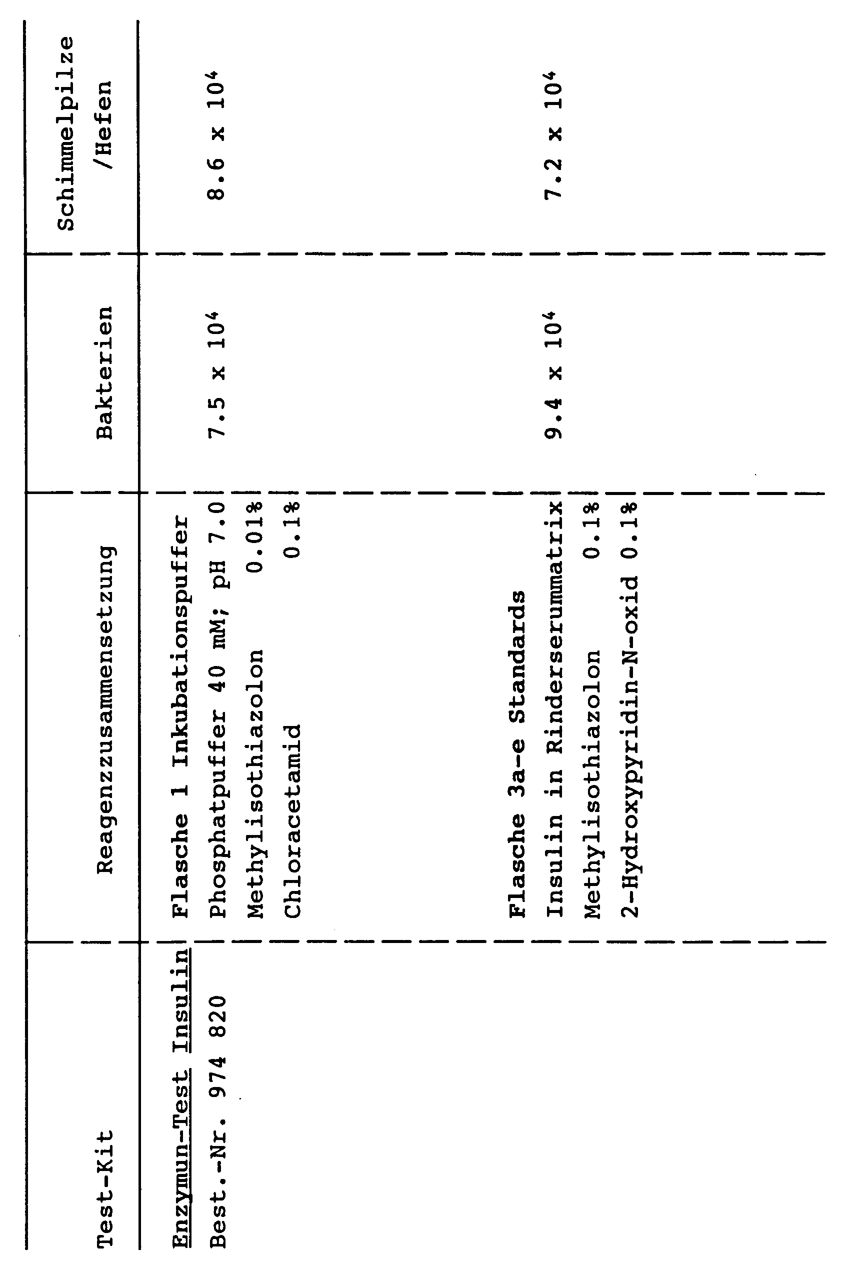

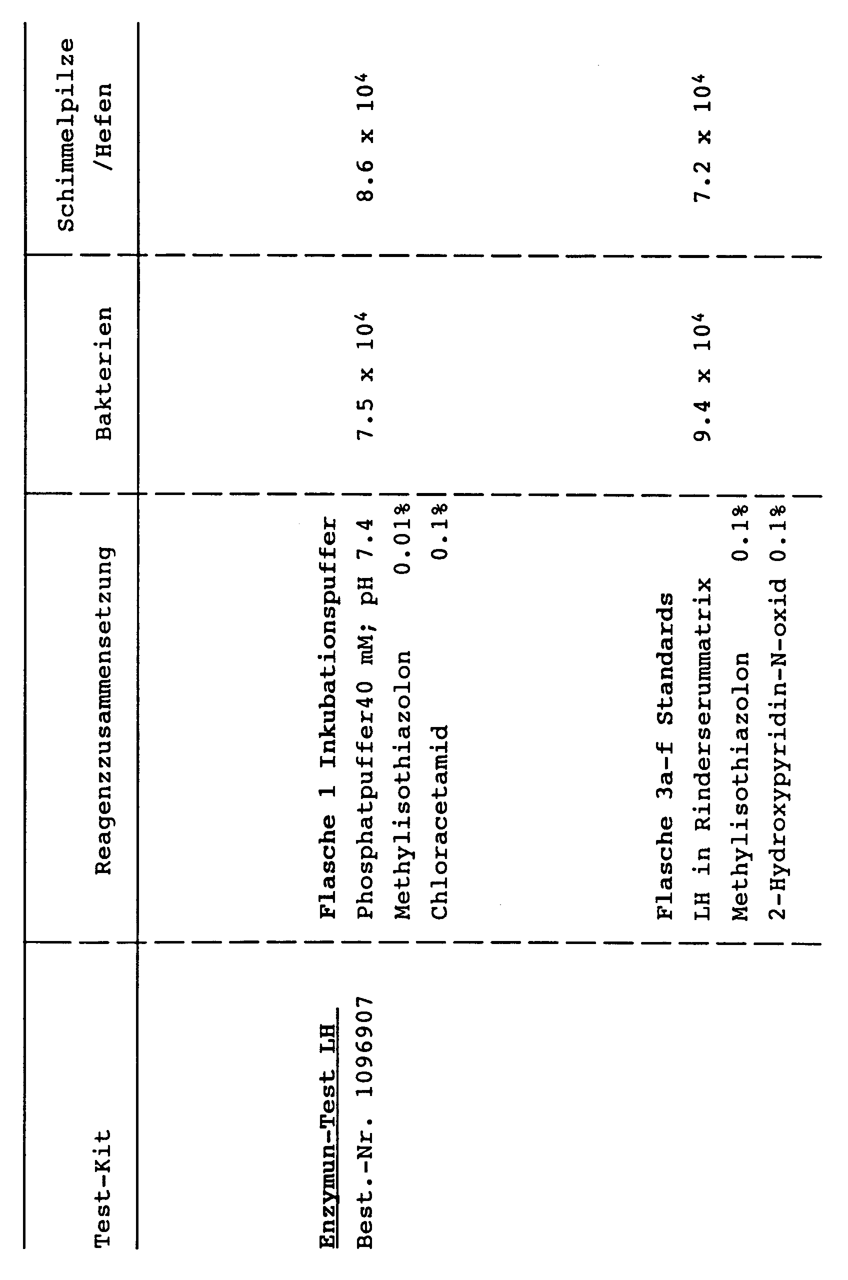

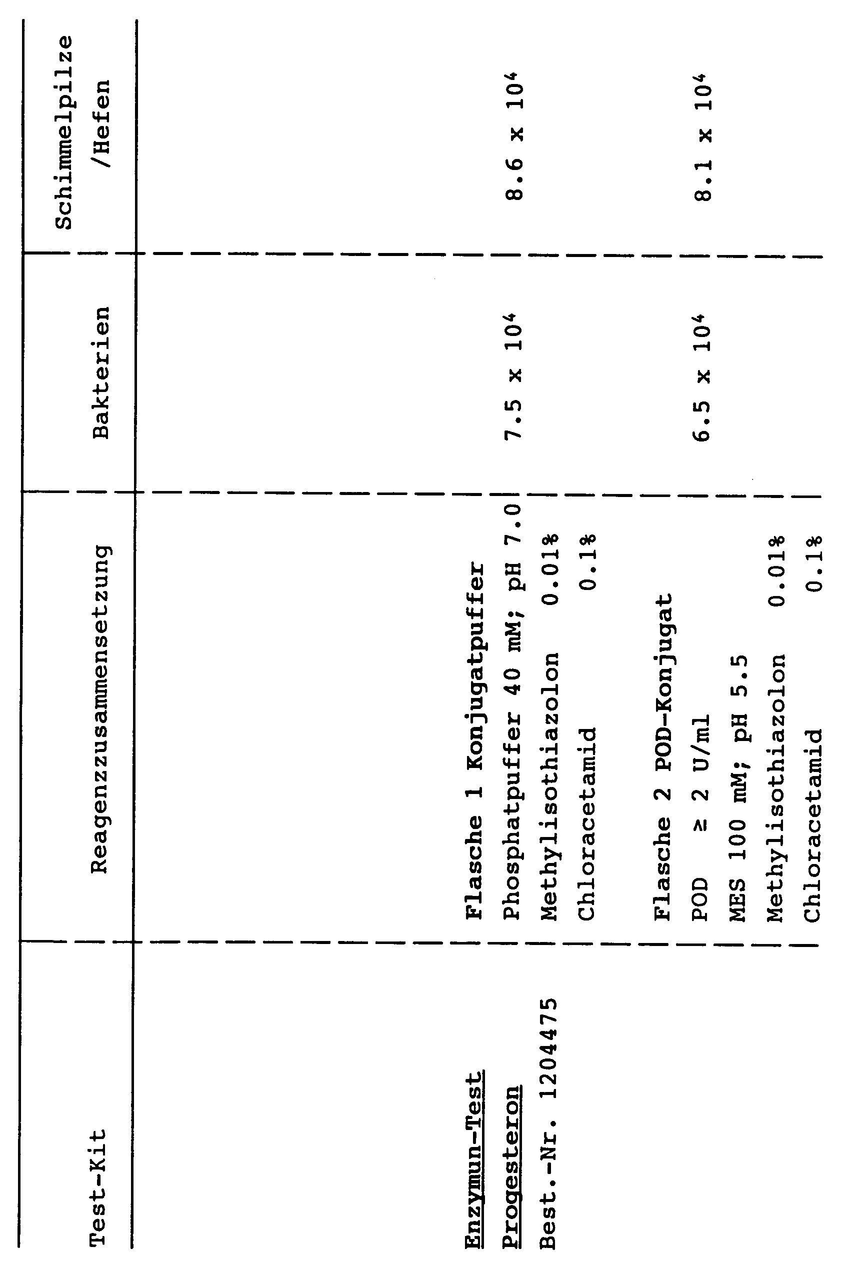

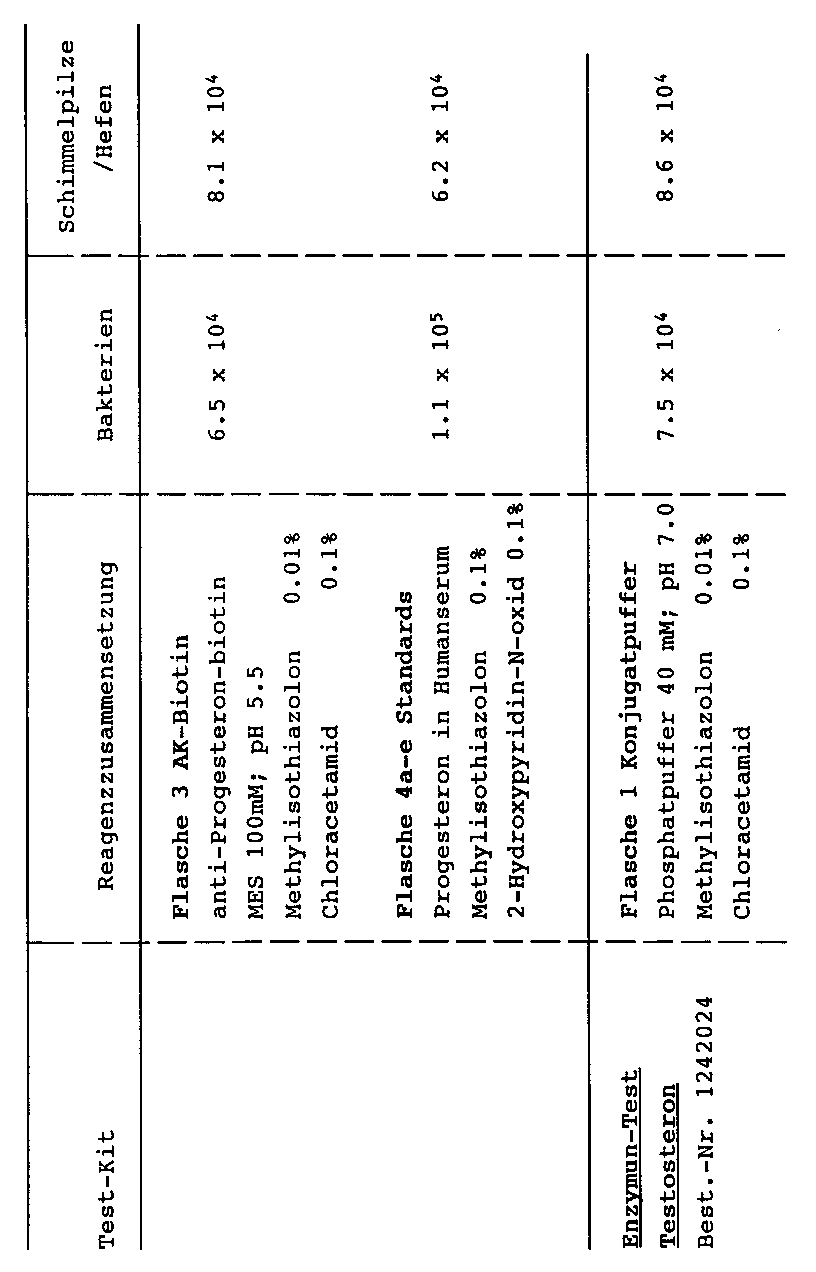

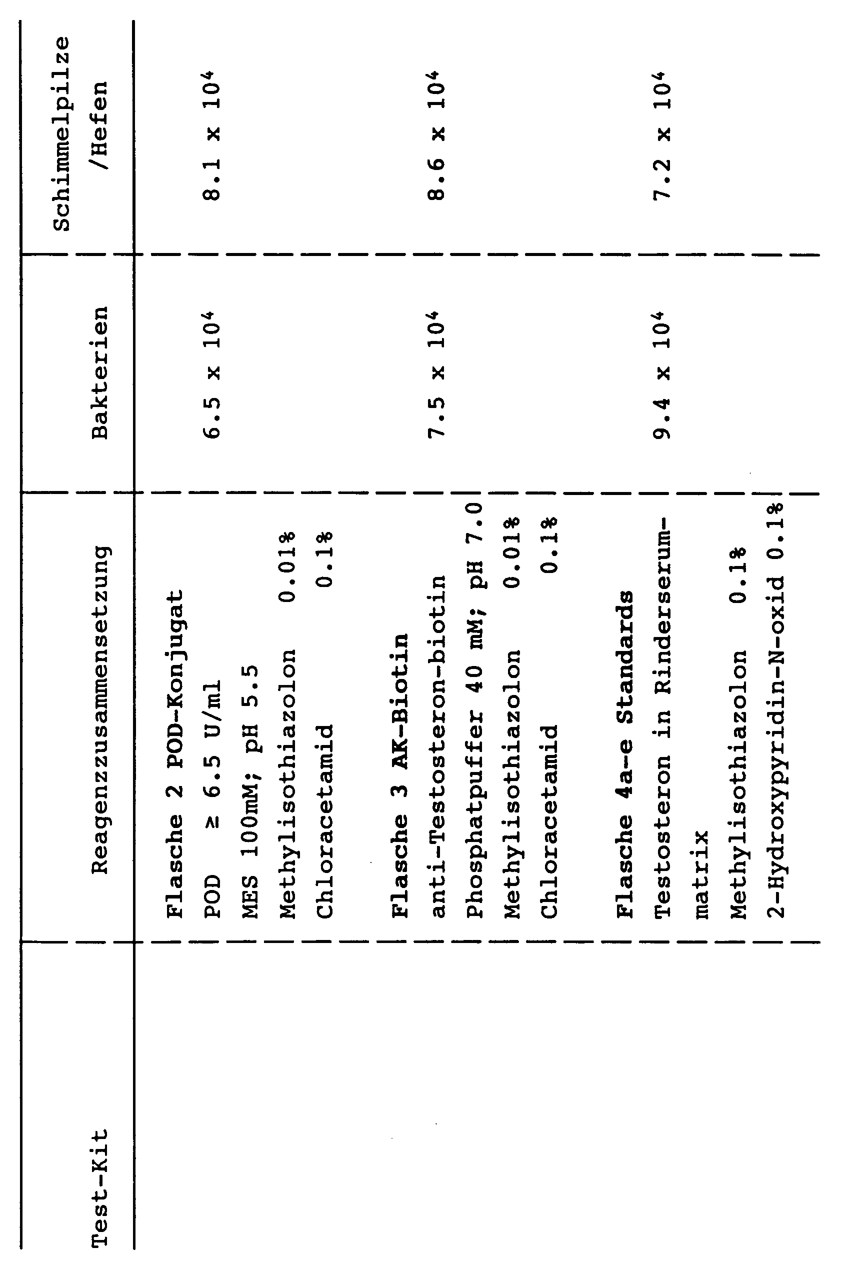

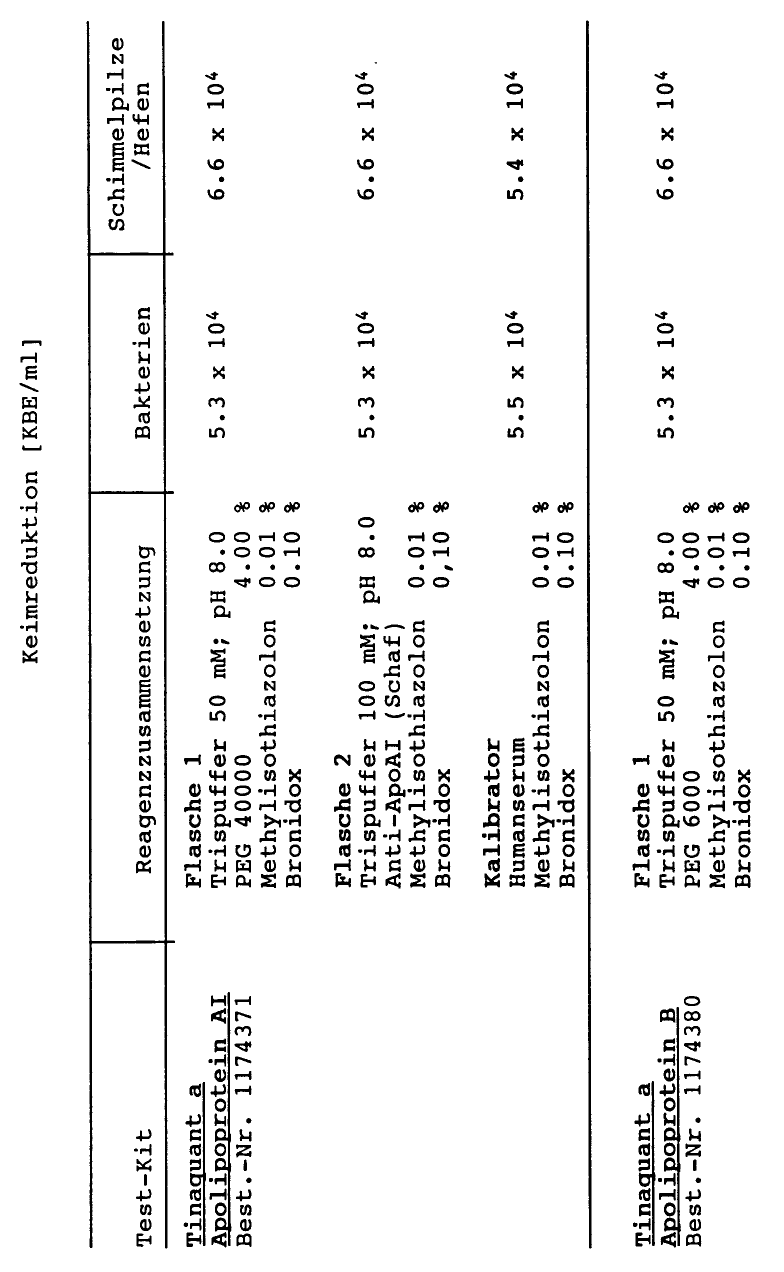

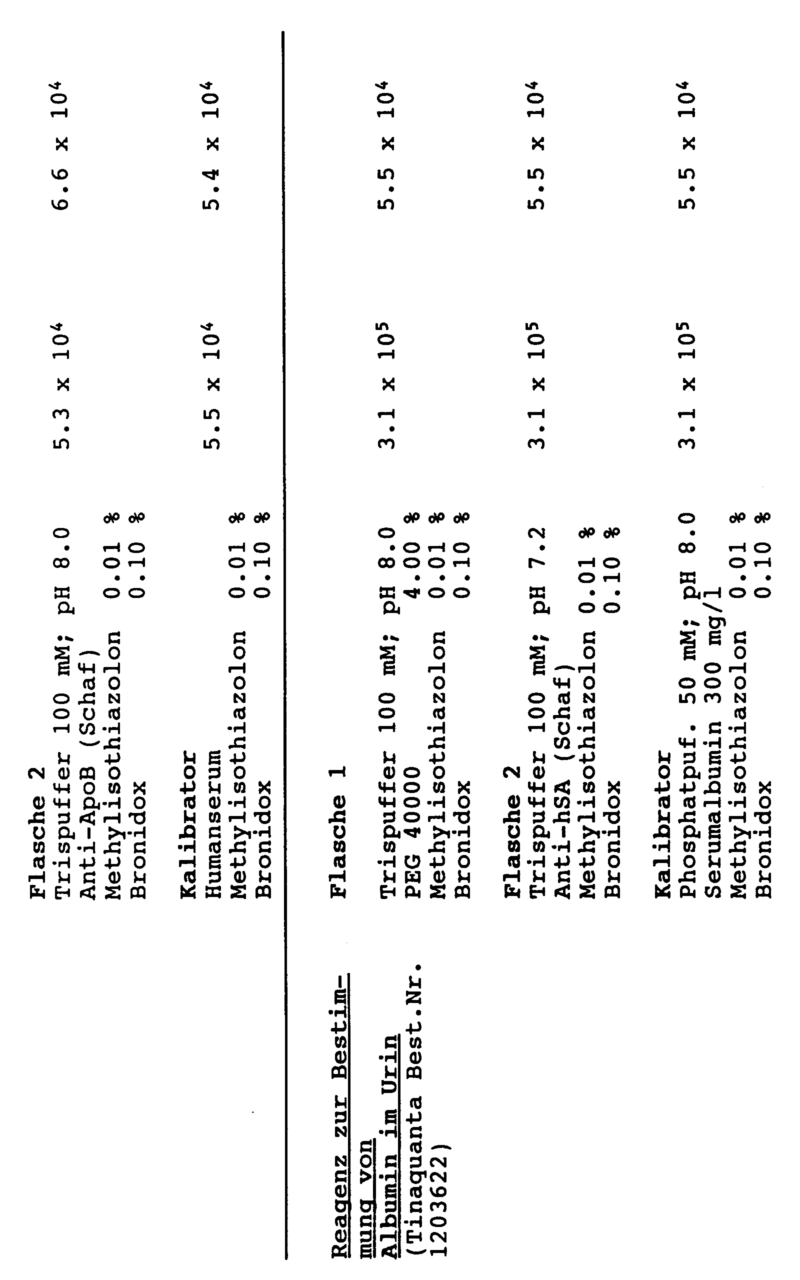

### 5. Keimreduktion:

5.1. Bakterien Die Koloniezahl soll im Laufe der Beobachtungszeit um mindestens 10³ KBE abnehmen, es darf keine signifikante Keimvermehrung auftreten.
5.2. Schimmelpilze/Hefen
   Die Koloniezahl soll im Laufe der Beobachtungszeit um mindestens 10² KBE abnehmen, es darf keine signifikante Keimvermehrung auftreten.

### 6. Beurteilung:

Die Konservierung ist ausreichend, wenn die Anforderungen nach Pkt. 5 erfüllt sind. In diesem Falle ist zu erwarten, daß die Schutzwirkung der Konservierung ausreichend ist gegen Kontamination, die bei Abfüllung unter unsterilen Bedingungen bzw. beim üblichen Umgang mit diesen Reagenzien auftreten (Luftkeime, Handkeime, unsterile Pipetten, unsterile Packmittel).

### 7. Einfluß auf die diagnostischen Testverfahren

Keine der verwendeten Kombinationen von Konservierungsmitteln zeigte einen Einfluß auf die Meßergebnisse der diagnostischen Tests nach Tabelle I.

## Patentansprüche (Patentansprüche für folgende(n) Vertragsstaat(en): AT, BE, CH, DK, ES, GR, LI, LU, SE)

1. Kombination zum Konservieren von diagnostischen Testkits,
**gekennzeichnet durch**
einen Gehalt an mindestens zwei Komponenten, ausgewählt aus der Gruppe, umfassend 2-Methyl-4-isothiazolin-3-on-hydrochlorid, 2-Hydroxypyridin-N-oxid, Chloracetamid, (N,N-methylen-bis(N-(1-hydroxymethyl)-2,5-dioxo-4-imidazolidinyl))-harnstoff und 5-Brom-5-nitro-1,3-dioxan, ausgenommen die Kombination von Chloracetamid mit (N,N-methylen-bis(N-1-hydroxymethyl)-2,5-dioxo-4-imidazolidinyl))-harnstoff.

2. Kombination nach Anspruch 1, **dadurch gekennzeichnet,** daß er 2-Methyl-4-isothiazolin-3-on-hydrochlorid enthält.

3. Konservierter diagnostischer Testkit, umfassend Testreagenzien und mindestens zwei Konservierungsmittel, ausgewählt aus der Gruppe, umfassend 2-Methyl-4-isothiazolin-3-on-hydrochlorid, 2-Hydroxypyridin-N-oxid, Chloracetamid, (N,N-methylen-bis(N-(1-hydroxymethyl)-2,5-dioxo-4-imidazolidinyl))-harnstoff und 5-Brom-5-nitro-1,3-dioxan enthält.

4. Testkit nach Anspruch 3,
**dadurch gekennzeichnet,**
daß er 2-Methyl-4-isothiazolin-3-on-hydrochlorid enthält.

5. Testkit nach Anspruch 3 oder 4,
**dadurch gekennzeichnet,**
daß er 2-Methyl-4-isothiazolin-3-on-hydrochlorid, in einer Wirkkonzentration von 0,001 bis 0,5 % enthält.

6. Testkit nach Anspruch 3 bis 5, **dadurch gekennzeichnet,** daß er 2-Hydroxypyridin-N-oxid in einer Wirkkonzentration von 0,1 bis 0,5 % enthält.

7. Testkit nach Anspruch 3 bis 6, **dadurch gekennzeichnet,** daß er Chloracetamid in einer Wirkkonzentration von 0,1 bis 0,5 % enthält.

8. Testkit nach Anspruch 3 bis 7, **dadurch gekennzeichnet,** daß er (N,N-methylen-bis(N-(1-hydroxymethyl)-2,5-dioxo-4-imidazolidinyl))-harnstoff in einer Wirkkonzentration von 0,1 bis 0,5 % enthält.

9. Testkit nach Anspruch 3 bis 7, **dadurch gekennzeichnet,** daß er 5-Brom-5-nitro-1,3-dioxan in einer Wirkkonzentration von 0,001 bis 0,5 % enthält.

10. Verwendung der Konservierungsmittel-Kombination nach Anspruch 1 oder 2 zur Konservierung von diagnostischen Testkits.

## Patentansprüche (Patentansprüche für folgende(n) Vertragsstaat(en): DE, FR, GB, IT, NL)

1. Kombination zum Konservieren von diagnostischen Testkits,
**gekennzeichnet durch**
einen Gehalt an mindestens zwei Komponenten, ausgewählt aus der Gruppe, umfassend 2-Methyl-4-isothiazolin-3-on-hydrochlorid, 2-Hydroxypyridin-N-oxid, Chloracetamid, (N,N-methylen-bis (N- (1-hydroxymethyl) -2, 5-dioxo-4-imidazolidinyl))-harnstoff und 5-Brom-5-nitro-1,3-dioxan, ausgenommen die Kombination von Chloracetamid mit (N,N-methylen-bis(N-1-hydroxymethyl)-2,5-dioxo-4-imidazolidinyl)-harnstoff und ausgenommen die Kombination von 2-Methyl-4-isothiazolin-3-on-hydrochlorid mit 5-Brom-5-nitro-1,3-dioxan.

2. Kombination nach Anspruch 1, **dadurch gekennzeichnet,** daß er 2-Methyl-4-isothiazolin-3-on-hydrochlorid enthält.

3. Konservierter diagnostischer Testkit, umfassend Testreagenzien und mindestens zwei Konservierungsmittel, ausgewählt aus der Gruppe, umfassend 2-Methyl-4-isothiazolin-3-on-hydrochlorid, 2-Hydroxypyridin-N-oxid, Chloracetamid, (N,N-methylen-bis(N-(1-hydroxymethyl)-2,5-dioxo-4-imidazolidinyl))-harnstoff und 5-Brom-5-nitro-1,3-dioxan enthält.

4. Testkit nach Anspruch 3,
**dadurch gekennzeichnet,**
daß er 2-Methyl-4-isothiazolin-3-on-hydrochlorid enthält.

5. Testkit nach Anspruch 3 oder 4,
**dadurch gekennzeichnet,**
daß er 2-Methyl-4-isothiazolin-3-on-hydrochlorid, in einer Wirkkonzentration von 0,001 bis 0,5 % enthält.

6. Testkit nach Anspruch 3 bis 5, **dadurch gekennzeichnet,** daß er 2-Hydroxypyridin-N-oxid in einer Wirkkonzentration von 0,1 bis 0,5 % enthält.

7. Testkit nach Anspruch 3 bis 6, **dadurch gekennzeichnet,** daß er Chloracetamid in einer Wirkkonzentration von 0,1 bis 0,5 % enthält.

8. Testkit nach Anspruch 3 bis 7, **dadurch gekennzeichnet,** daß er (N,N-methylen-bis(N-(1-hydroxymethyl)-2,5-dioxo-4-imidazolidinyl))-harnstoff in einer Wirkkonzentration von 0,1 bis 0,5 % enthält.

9. Testkit nach Anspruch 3 bis 7, **dadurch gekennzeichnet,** daß er 5-Brom-5-nitro-1,3-dioxan in einer Wirkkonzentration von 0,001 bis 0,5 % enthält.

10. Verwendung der Konservierungsmittel-Kombination nach Anspruch 1 oder 2 zur Konservierung von diagnostischen Testkits.

## Claims (Claims for the following Contracting State(s): AT, BE, CH, DK, ES, GR, LI, LU, SE)

1. Combination for the preservation of diagnostic tests, characterised by a content of at least two components selected from the group comprising 2-methyl-4-isothiazolin-3-one hydrochloride, 2-hydroxypyridine-N-oxide, chloroacetamide, (N,N-methylene-bis-(N-(1-hydroxymethyl)-2,5-dioxo-4-imidazolidinyl))-urea and 5-bromo-5-nitro-1,3-dioxane, excluding the combination of chloroacetamide with (N,N-bis-(N-1-hydroxymethyl)-2,5-dioxo-4-imidazolidinyl)-urea.

2. Combination according to claim 1, characterised in that it contains 2-methyl-4-isothiazolin-3-one hydrochloride.

3. Preserved diagnostic test kit, comprising test reagents and at least two preservation agents selected from the group comprising 2-methyl-4-isothiazolin-3-one hydrochloride, 2-hydroxypyridine-N-oxide, chloroacetamide, (N,N-methylene-bis-(N-(1-hydroxymethyl)- 2,5-dioxo-4-imidazolidinyl))-urea and 5-bromo-5-nitro-1,3-dioxane.

4. Test kit according to claim 3, characterised in that it contains 2-methyl-4-isothiazolin-3-one hydrochloride.

5. Test kit according to claim 3 or 4, characterised in that it contains 2-methyl-4-isothiazolin-3-one hydrochloride in an active concentration of 0.001 to 0.5%.

6. Test kit according to claim 3 to 5, characterised in that it contains 2-hydroxypyridine-N-oxide in an active concentration of 0.1 to 0.5%.

7. Test kit according to claim 3 to 6, characterised in that it contains chloroacetamide in an active concentration of 0.1 to 0.5%.

8. Test kit according to claim 3 to 7, characterised in that it contains (N,N-methylene-bis-(N-(1-hydroxymethyl)-2,5-dioxo-4-imidazolidinyl))-urea in an active concentration of 0.1 to 0.5%.

9. Test kit according to claim 3 to 7, characterised in that it contains 5-bromo-5-nitro-1,3-dioxane in an active concentration if 0.001 to 0.5%.

10. Use of the preservation agent combination according to claim 1 or 2 for the preservation of diagnostic test kits.

## Claims (Claims for the following Contracting State(s): DE, FR, GB, IT, NL)

1. Combination for the preservation of diagnostic tests, characterised by a content of at least two components selected from the group comprising 2-methyl-4-isothiazolin-3-one hydrochloride, 2-hydroxypyridine-N-oxide, chloroacetamide, (N,N-methylene-bis-(N-(1-hydroxymethyl)-2,5-dioxo-4-imidazolidinyl))-urea and 5-bromo-5-nitro-1,3-dioxane, excluding the combination of chloroacetamide and (N,N-methylene-bis-(N-1-hydroxymethyl)-2,5-dioxo-4-imidazolidinyl)-urea and excluding the combination of 2-methyl-4-isothiazolin-3-one hydrochloride with 5-bromo-5-nitro-1,3-dioxane.

2. Combination according to claim 1, characterised in that it contains 2-methyl-4-isothiazolin-3-one hydrochloride.

3. Preserved diagnostic test kit, comprising test reagents and at least two preservation agents selected from the group comprising 2-methyl-4-isothiazolin-3-one hydrochloride, 2-hydroxypyridine-N-oxide, chloroacetamide, (N,N-methylene-bis-(N-(1-hydroxymethyl)-2,5-dioxo-4-imidazolidinyl))-urea and 5-bromo-5-nitro-1,3-dioxane.

4. Test kit according to claim 3, characterised in that it contains 2-methyl-4-isothiazolin-3-one hydrochloride.

5. Test kit according to claim 3 or 4, characterised in that it contains 2-methyl-4-isothiazolin-3-one hydrochloride in an active concentration of 0.001 to 0.5%.

6. Test kit according to claim 3 to 5, characterised in that it contains 2-hydroxypyridine-N-oxide in an active concentration of 0.1 to 0.5%.

7. Test kit according to claim 3 to 6, characterised in that it contains chloroacetamide in an active concentration of 0.1 to 0.5%.

8. Test kit according to claim 3 to 7, characterised in that it contains (N,N-methylene-bis-(N-(1-hydroxymethyl)-2,5-dioxo-4-imidazolidinyl))-urea in an active concentration of 0.1 to 0.5%.

9. Test kit according to claim 3 to 7, characterised in that it contains 5-bromo-5-nitro-1,3-dioxane in an active concentration of 0.001 to 0.5%.

10. Use of the preservation combinations according to claim 1 or 2 for the preservation of diagnostic test kits.

## Revendications (Revendications pour l'(les) Etat(s) contractant(s) suivant(s): AT, BE, CH, DK, ES, GR, LI, LU, SE)

1. Combinaison pour la conservation de trousses de tests diagnostiques, caractérisée par une teneur en au moins deux constituants choisis dans le groupe comprenant le chlorhydrate de 2-méthyl-4-isothiazolin-3-one, le 2-hydroxypyridine-N-oxyde, le chloracétamide, la (N,N-méthylène-bis(N-(1-hydroxyméthyl)-2,5-dioxo-4-imidazolidinyl))-urée et le 5-bromo-5-nitro-1,3-dioxane, à l'exception de la combinaison du chloracétamide avec la (N,N-méthylène-bis(N-(1-hydroxyméthyl)-2,5-dioxo-4-imidazolidinyl))-urée.

2. Combinaison selon la revendication 1, caractérisée en ce qu'elle contient du chlorhydrate de 2-méthyl-4-isothiazolin-3-one.

3. Trousse de test diagnostique conservée, comprenant des réactifs de test et au moins deux agents de conservation choisis dans le groupe comprenant le chlorhydrate de 2-méthyl-4-isothiazolin-3-one, le 2-hydroxypyridine-N-oxyde, le chloracétamide, la (N,N-méthylène-bis(N-(1-hydroxyméthyl)-2,5-dioxo-4-imidazolidinyl))-urée et le 5-bromo-5-nitro-1,3-dioxane.

4. Trousse de test selon la revendication 3, caractérisée en ce qu'elle contient du chlorhydrate de 2-méthyl-4-isothiazolin-3-one.

5. Trousse de test selon la revendication 3 ou 4, caractérisée en ce qu'elle contient du chlorhydrate de 2-méthyl-4-isothiazolin-3-one en une concentration efficace de 0,001 à 0,5%.

6. Trousse de test selon les revendications 3 à 5, caractérisée en ce qu'elle contient du 2-hydroxypyridine-N-oxyde en une concentration efficace de 0,1 à 0,5%.

7. Trousse de test selon les revendications 3 à 6, caractérisée en ce qu'elle contient du chloracétamide en une concentration efficace de 0,1 à 0,5%.

8. Trousse de test selon les revendications 3 à 7, caractérisée en ce qu'elle contient de la (N,N-méthylène-bis(N-(1-hydroxyméthyl)-2,5-dioxo-4-imidazolidinyl)-urée en une concentration efficace de 0,1 à 0,5%.

9. Trousse de test selon les revendications 3 à 7, caractérisée en ce qu'elle contient du 5-bromo-5-nitro-1,3-dioxane en une concentration efficace de 0,001 à 0,5%.

10. Utilisation de la combinaison d'agents de conservation selon la revendication 1 ou 2 pour la conservation de trousses de tests diagnostiques.

## Revendications (Revendications pour l'(les) Etat(s) contractant(s) suivant(s): DE, FR, GB, IT, NL)

1. Combinaison pour la conservation de trousses de tests diagnostiques, caractérisée par une teneur en au moins deux constituants choisis dans le groupe comprenant le chlorhydrate de 2-méthyl-4-isothiazolin-3-one, le 2-hydroxypyridine-N-oxyde, le chloracétamide, la (N,N-méthylène-bis(N-(1-hydroxyméthyl)-2,5-dioxo-4-imidazolidinyl))-urée et le 5-bromo-5-nitro-1,3-dioxane, à l'exception de la combinaison du chloracétamide avec la (N,N-méthylène-bis(N-(1-hydroxyméthyl)-2,5-dioxo-4-imidazolidinyl))-urée et à l'exception de la combinaison du chlorhydrate de 2-méthyl-4-isothiazolin-3-one avec le 5-bromo-5-nitro-1,3-dioxane.

2. Combinaison selon la revendication 1, caractérisée en ce qu'elle contient du chlorhydrate de 2-méthyl-4-isothiazolin-3-one.

3. Trousse de test diagnostique conservée, comprenant des réactifs de test et au moins deux agents de conservation choisis dans le groupe comprenant le chlorhydrate de 2-méthyl-4-isothiazolin-3-one, le 2-hydroxypyridine-N-oxyde, le chloracétamide, la (N,N-méthylène-bis(N-(1-hydroxyméthyl)-2,5-dioxo-4-imidazolidinyl))-urée et le 5-bromo-5-nitro-1,3-dioxane.

4. Trousse de test selon la revendication 3, caractérisée en ce qu'elle contient du chlorhydrate de 2-méthyl-4-isothiazolin-3-one.

5. Trousse de test selon la revendication 3 ou 4, caractérisée en ce qu'elle contient du chlorhydrate de 2-méthyl-4-isothiazolin-3-one en une concentration efficace de 0,001 à 0,5%.

6. Trousse de test selon les revendications 3 à 5, caractérisée en ce qu'elle contient du 2-hydroxypyridine-N-oxyde en une concentration efficace de 0,1 à 0,5%.

7. Trousse de test selon les revendications 3 à 6, caractérisée en ce qu'elle contient du chloracétamide en une concentration efficace de 0,1 à 0,5%.

8. Trousse de test selon les revendications 3 à 7, caractérisée en ce qu'elle contient de la (N,N-méthylène-bis(N-(1-hydroxyméthyl)-2,5-dioxo-4-imidazolidinyl)-urée en une concentration efficace de 0,1 à 0,5%.

9. Trousse de test selon les revendications 3 à 7, caractérisée en ce qu'elle contient du 5-bromo-5-nitro-1,3-dioxane en une concentration efficace de 0,001 à 0,5%.

10. Utilisation de la combinaison d'agents de conservation selon la revendication 1 ou 2 pour la conservation de trousses de tests diagnostiques.
